# EUROPEAN PATENT APPLICATION

(11) **EP 1 897 531 A1**
(43) Date of publication of application: **12.03.2008**
(21) Application number: 06018937.0
(22) Date of filing: 11.09.2006
(51) Int. Cl.: A61K 8/41, A61K 8/49, A61Q 5/10

(54) **Hair colouring composition**

(71) Applicant: KPSS Kao Professional Salon Services GmbH, 64297 Darmstadt (DE)
(72) Inventor: Uellner, Martin, 64295 Darmstadt (DE); Kufner, Frank, 64297 Darmstadt (DE); Blumenschein, Katrin, 64753 Brombachtal-Hembach (DE)

(57) **Abstract**

Present invention relates to a composition for oxidative colouring hair especially chemically multiple processed hair and more specifically at least twice bleached hair.

## Description

Present invention relates to a composition for oxidative colouring hair especially chemically multiple processed hair and more specifically at least twice bleached hair.

Bleaching and oxidative colouring hair is a common practice in hair dressing area. Especially the female users of hair colouring products tend to change their hair colour considerably often and time to time present various wishes which gives or can give certain difficulties. Such difficulties are especially observed when hair is considerably damaged by previous or in the same process right before oxidative colouring hair performed chemical treatments especially bleaching treatments. One of them is difficulty in achieving so called warm shades with oxidative colouring hair. With the term "warm shade" it is meant that hair colour thus obtained comprises a red, reddish touch. In other words when a hair colour is expressed with its "L", "a" and "b" values as measured by a laboratory colour measuring device wherein "L" being the intensity of the colour, "a" being the intensity of red colour and "b" being the intensity of the yellow colour, both "a" and "b" values are having a positive value independent from the colour intensity,

In practice when colouring hair especially multiple processed hair and more specifically at least twice bleached hair with an oxidative dyeing compositions into a warm shade the resulting colour is often not satisfactorily warm enough. In other words, the colour obtained does not have red or reddish touch as expressed with a positive "a" and "b" value, In order to overcome such results, obtained colours either have to be corrected by means of application of another colouring composition, or application of a pre-colouration composition as well comprising hair colorants either oxidative or direct, or mixing colouring composition with an another colouring composition prior to application. All of the mentioned options are currently being the usual practice at hair dressing salons, Even the latter one has further drawbacks as it is solely based on the hair dresser's colour analysis.

Furthermore, it has also been observed that colours obtained lack of homogeneity as roots and tips having different colour intensity. Moreover, stability of colours so obtained especially against shampooing is varying largely, even from root to tip of the hair.

The aim of the present invention is to overcome above mentioned problems and provide a oxidative hair colouring composition for colouring hair especially multiple processed hair and more specifically at least twice bleached hair into a warm shade in a single process without application of any pre-treatment or preparation of new colour mixtures by mixing two dyestuff containing colouring compositions prior to application.

The present inventors have surprisingly found out that a composition for oxidative colouring hair comprising at least one oxidative dye precursor and at least one coupling substance colours hair into a warm colour, i.e. colur with a red and/or reddish touch, wherein "a" and "b" values measured with a colour measuring device of the hair tress after colouring are both positive and "a" is at least 80%, preferably equal to or higher than that of the "b" value characterised in that the alkaline content is below or equal to 2.5% by weight, expressed as ammonia content, measured prior to mixing with an oxidizing agent.

Alkaline content of the composition is preferably below or equal to 2% by weight and more preferably takes the values between 0.05 and 0.50 and even more preferrably between 0.1 and 0.25% by weight calculated to total composition, expressed as ammonia content. For achieving the alkaline value any know alkalizer can be used. Special mention is made to ammonium hydroxide and monoethanolamine.

The "a" and "b" values as obtained by measuring the colour of the tresses after colouring with a laboratory equipment is positive and "a" value is at least 80% of the "b" value, preferably at least has the same number as the "b" value or higher (meaning at least 100% of the "a" value) and more preferably higher than "b" value (meaning more than 100) at any given colour intensity ("L" value).

The effects of the present invention are better observed when colouring multiple processed hair, i.e. subjected more than once to a chemical treatment and at the best observed on a hair bleached at least twice or more. Accordingly, the subject matter of the present invention is oxidative colouring multiple processed and/or at least twice or more bleached hair into a warm colour direction wherein "a" value as measured with a laboratory device after colouring hair with oxidative colouring composition is at least 80% of the "b" value at any "L" value characterised in that the alkaline content is below or equal to 2.5% by weight calculated to total composition, expressed as ammonia content, measured prior to mixing with an oxidizing agent.

Still further objective of the present invention is use of a composition comprising at least one oxidative dye precursor and at least one coupler and having an alkaline content of below or equal to 2.5%, preferably below or equal to 2.0%, more preferably 0.05 to 0.5% and in particular 0.1 to 0.25% by weight expressed as ammonia content calculated to total composition prior to mixing with an oxidizing agent for colouring hair and especially multiple processed hair and in particular at least twice bleached hair into a warm colour directions wherein "a" value is at least 80% of the "b" value, preferably at least has the same number as the "b" value and more preferably is higher than "b" value at any "L" value

Composition of the present invention comprises at least one oxidative dye precursor. Some examples are p-phenlynediamine, p-methyleminophenol and substituted p-phenylenediamines such as 2,5-diamino-toluene, 2-n-propyl or 2-ethyl-p-phenylenediamine, 2,6-di-methyl-p-phenylene- dlamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

Preferred are p-toluenediamine and salts thereof, 2,5,6-triamino-4-pyrimidine and salts thereof, In a further preferred embodiment of the present invention the p-toluenediamine and salts thereof, 2,5,6-triamino-4-pyrimidinol and salts thereof are contained together in the same composition.

The composition according to the invention preferably comprises at least one coupling substance. Suitable ones are resorcinol, 2-methyl resorcinol, 4-chlororesorcinol, 2-amino-4-chlorophenol, 5-amino-4-methoxy-2-methylphenal, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5,aminopyridine, 2,6-diaminopyridine, 1,3-diaminobenzene, 1-amino-3-(2'-hy-droxyethylamino)benzene, 1-amino-3-[bis(2'-hydroxyethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol or the water-soluble salts thereof.

Preferred are resorcinol, m-aminophenol, 2-amino-3hydroxypyridine, 4-amino-m-cresol and mixtures thereof. In a preferred embodiment of the present invention the compositions of the present invention comprises at least 2 of the aforementioned coupling substances.

Total concentration of oxidative dye precursors and coupling substances are in the range of 0.01 to 10% by weight, preferably 0.05 to 7.5% by weight and more preferably 0.1 to 5% by weight, calculated to total composition prior to mixing with an oxidizing agent. Weight ratio of one or more oxidative dye precursors to one or more coupler substance is in the range of 1:8 to 8:1, preferably 1:5 to 5:1 and more preferably 1:2 to 2:1.

Compositions of the present invention may also comprise direct dyes. It should be noted that the positive "a" and "b" values of the defined colour is already observed before addition of any direct dye. Direct dyes are found to be useful for fine tuning any colour tone within the meaning of the present invention.

Direct dyes can be cationic, anionic and/or nitro dyes, Some examples to cationic ones are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green, Basic Orange 31, 1, Basic Red 2, Basic Red 12 Basic Red 22, Basic Red 76 Basic Red 51, Basic Violet 1, Basic Violet 2, Basic Violet 3, Basic Violet 10, Basic Violet 14 and Basic Yellow 57 Basic Yellow 87.

Examples to anionic ones are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No, 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1. HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No,54, HC Red No.14, HC Violet BS. HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15,2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of direct dyes is as a rule low. However, higher amounts is not excluded, as it is mentioned above within the meaning of the present invention the positive "a" and "b" values and an "a" value of at least 80% of the "b" value is achieved before addition of the direct dyes. Although this, it should be seen that compositions comprising direct dyes as a total if there are more than one direct dye present less that 10% of the total oxidative dyes being bases and coupler is still understood to be within the meaning of the present invention as the addition of direct dye is not for altering the "a" and "b" but rather fine tuning of the shade. pH of the composition is in the range of 5 to 12, preferably 6 to 11 and more preferably 6.8 to 10, in particular 6.8 to 7 after mixing with an oxidizing agent.

Compositions of the present invention is mixed prior to application onto hair with a composition comprising at least one oxidizing agent. The preferred oxidizing agent is hydrogen peroxide at a concentration of 2 to 12% by weight. Other peroxides such as urea peroxide, and melanin peroxide is also possible to use.

At the same time, the subject of the present invention is process for colouring chemically multiple processed hair especially at least twice bleached hair by applying a composition comprising at least one oxidative dye precursor and at least one coupling substance and having an alkaline value of below or equal to 2.5% by weight expressed as ammonia content, preferably equal or less than 2.0% by weight and more preferably between 0.05 and 0,5% and still more preferably between 0.1 and 0.25% mixed with a composition comprising at least one oxidizing agent, preferably hydrogen peroxide and after processing time of 15 to 45 min at a temperature of 20 to 45°C rinsed off from hair wherein the obtained colour has "a" and "b" values, as measured with a colour measuring device, both positive and "a" value is at least 80% of the "b" value, preferably same as the "b" value or higher at any colour intensity.

Oxidative colouring compositions of the present invention can be in the form of a gel, solution, dispersion or emulsion. The most preferred form is the emulsion form.

Accordingly, oxidative colouring compositions of the present invention can comprise all other ingredients customarily found in a composition designed therefore.

Such compounds are for example surfactants of anionic, cationic, non-ionic and/or amphoteric types or their mixtures. Examples are fatty alcohol ether sulfates, fatty alcohol sulfates, fatty alcohol ether carboxylates as anionic surfactants, alkyl glucosides, ethoxylated fatty alcohols, fatty acids alanol amides as non-ionic surfactants, mono or dialkyl quaternary ammonium surfactants, ester and amido group containing quaternary ammonium surfactants as cationic surfactants and exaples to the amphoteric ones are alkyl betaines, sultaines and especially the amido betaines such as cocamidopropylbetaine.

In the case that the composition is in form of an emulsion, preferred form, composition comprise as a rule fatty alcohol with a chain length of 14 to 22 C atoms. The preferred are myristyl, cetyl, cetearyl, stearyl and behenyl alcohols. Among those cetearyl alcohol which is the mixture of cetyl and stearyl alcohols is the most preferred.

Fatty acids can be part of the compositions such as palmitic, stearic, oleic acid. Oleic acid is the most preferred one.

Polymeric thickeners, solvents, preservatives antioxidants, fragrances, reducing agents, chelating agents can be added as usually found in oxidative colouring compositions.

Following example should illustrate the invention.

### Example

| | % by weight |
|---|---|
| Cetearyl alcohol | 10.0 |
| Propylene glycol | 4.0 |
| Ceteareth-30 | 2.0 |
| Laureth-2 | 0.5 |
| Behentrimonium chloride | 2.0 |
| Panthenol | 0.5 |
| Sodium sulfit | 0.5 |
| Ascorbic acid | 0.2 |
| Tetrasodium EDTA | 0.2 |
| Fragrance, preservative | q.s |
| Ethanolamine | 0.3 |
| p-toluenediamine sulfate | 0.43 |
| 2,5,6-triamino-4-pyrimidinol sulfate | 0.01 |
| Resorcinol | 0.01 |
| m-aminophenol | 0.07 |
| 2-amino-3-hydroxypyridine | 0.03 |
| 4-amino-m-cresol | 0,02 |
| Water | q.s. to 100 |

The above composition has a alkaline content of 0.2% by weight expressed as ammonia content calculated to total composition and has a viscosity of approximately 9000 mPa.s measured at 20°C with a Brookfield viscosimetre at a shear rate of 10 s⁻¹, and has a pH of 9.5.

The above composition was mixed with a composition comprising hydrogen peroxide at a concentration of 2% by weight at a weight ratio of 1:1 which was resulted in a pH value of 6,9 and was applied onto a twice bleached hair. After processing of 30 min at 30°0 the hair was rinsed with tap water and shampooed once with a commercial shampoo and dried with a hair drier. The following "L", "a" and "b" values were measured with a laboratory equipment supplied by Minolta.

| | | |
|---|---|---|
| L; 23.17 | a:7.02 | b:6.35 (the values are mean of 3 measurements). |

A shiny warm homogeneous colour was obtained. The L, a and b values measured at the root and tip parts as identified on the tress used were not significantly differing from the above mentioned values.

## Claims

1. Composition for oxidative colouring hair, especially chemically multiple treated hair and more specifically at least twice bleached hair into a warm shade, said warm colour shade including red or reddish touch which is a colour **characterised by** its "L" value for its intensity and "a" value showing intensity of red and "b" value showing intensity of yellow wherein both "a" and "b" value are positive independent from the colour intensity and "a" value is at least 80% of the "b" value comprising at least one oxidative dye precursor and at least one coupling substance **characterised in that** it has a alkaline content of 2.5% or less by weight expressed by ammonia content calculated to total content prior to mixing with an oxidizing agent.

2. Composition according to claim 1 wherein the alkaline content is between 0.05 and 0.5% by weight expressed by ammonia content calculated to total content prior to mixing with an oxidizing agent.

3. Composition according to claims 1 and 2 wherein the colour has "a" value having the same number as the "b" value or higher.

4. Composition according to any of the preceding claims **characterised in that** the oxidation dye precursor is selected from p-toluenediamine and 2,5,6,-triamino-4-pyrimidinol and their salts.

5. Composition according to any of the preceding claims **characterised in that** it comprises as oxidation dye precursor p-toluenediamine and/or its salts and 2,5,6,-triamino-4-pyrimidine and/or salts.

6. Composition according to any of the preceding claims **characterised in that** it comprises at least one coupling substance selected from resorcinol, m-aminophenol, 2-amino-3-hydroxypyridine, 4-amino-m-cresol.

7. Composition according to any claim 6 **characterised in that** it comprises at least two coupling substances selected from resorcinol, m-aminophenol, 2-amino-3-hydroxypyridine, 4-amino-m-cresol.

8. Composition according to any of the preceding claims **characterised in that** it has a pH between 5 and 12 after mixing with an oxidizing agent.

9. Process for colouring hair especially chemically multiple treated hair and more specifically at least twice bleached hair into a warm shade said warm colour shade including red or reddish touch which is a colour **characterised by** its "L" value for its intensity and "a" value showing intensity of red and "b" value showing intensity of yellow **characterised in that** a composition according to claims 1 to 8 is applied onto hair after mixing with a composition comprising at least one oxidizing agent, preferably hydrogen peroxide, and rinsed off after processing 15 to 45 min at a temperature between 20 and 45°C.

10. Use of a composition comprising at least one oxidative dye precursor and at least one coupling agent and having an alkaline content of 2.5% or less by weight expressed as ammonia content before mixing with an oxidizing agent for colouring hair especially chemically multiple treated hair and more specifically at least twice bleached hair into a warm shade, said warm colour shade including red or reddish touch which is a colour **characterised by** its "L" value for its intensity and "a" value showing intensity of red and "b" value showing intensity of yellow wherein both "a" and "b" value are positive independent from the colour intensity and "a" value is at least 80% of the "b" value
